# EUROPEAN PATENT APPLICATION

(11) **EP 4 282 399 A1**
(43) Date of publication of application: **29.11.2023**
(21) Application number: 22742647.5
(22) Date of filing: 20.01.2022
(51) Int. Cl.: A61K 8/68, A61K 31/164, A61Q 19/00, A61P 17/00, A61P 17/06, A61K 8/63, A61K 31/575, A61K 8/31, A61K 31/01, A61K 8/37, A61K 31/231, A61K 8/55, A61K 31/685, A61K 8/36, A61K 31/20, A61K 31/201, A61K 31/202

(54) **COSMETIC COMPOSITION**

(30) Priority: 20.01.2021 JP 2021006810
(71) Applicant: AJINOMOTO CO., INC., Chuo-ku Tokyo 104-8315 (JP)
(72) Inventor: HIGASHIURA, Eri, Kawasaki-shi, Kanagawa 210-8681 (JP); MORI, Kenichi, Kawasaki-shi, Kanagawa 210-8681 (JP); TAKINO, Yoshinobu, Kawasaki-shi, Kanagawa 210-8681 (JP)
(74) Representative: Strehl Schübel-Hopf & Partner
(86) International application number: PCT/JP2022/001984
(87) International publication number: WO 2022/158525

(57) **Abstract**

A composition, such as a cosmetic composition, is provided. A composition containing ceramide NH having a length of acyl chain (an acyl chain length) of C18.

## Description

### [Technical Field]

The present invention relates to a composition, such as a cosmetic composition.

### [Background Art]

A ceramide is a compound having a structure in which a sphingoid base and a fatty acid is covalently linked to each other via an amide bond. That is, a ceramide comprises a sphingoid base moiety (namely, an alkyl chain) and a fatty acid moiety (namely, an acyl chain), which moieties are covalently linked to each other via an amide bond. Various groups of ceramides have been known depending on the types of the sphingoid base and fatty acid (Non-patent document 1). Some ceramides have been used as ingredients for pharmaceuticals, cosmetics, and so forth.

Ceramide NH is a ceramide of 6-hydroxysphingosine (hereafter, also referred to as "6HS". That is, ceramide NH is a ceramide of which the sphingoid base moiety is 6HS. That is, ceramide NH comprises a 6HS moiety (namely, an alkyl chain) and a fatty acid moiety (namely, an acyl chain), which moieties are covalently linked to each other via an amide bond. As ceramide NHs, those having different lengths of acyl chains such as C18 have been known (Non-patent documents 1 and 2).

However, effects of lengths of acyl chains on functions of ceramide NHs have not been known.

### [Citation List]

### [Non-patent Literature]

[Non-patent document 1]
   Masukawa Y. et al., J Lipid Res. 2008 Jul;49(7):1466-76. Characterization of overall ceramide species in human stratum corneum.
[Non-patent document 2]
   Vielhaber G. et al., J Invest Dermatol. 2001 Nov;117(5):1126-36. Localization of ceramide and glucosylceramide in human epidermis by immunogold electron microscopy.

### [Summary of Invention]

### [Object to be Achieved by the Invention]

An object of the present invention is to provide a composition, such as a cosmetic composition.

### [Means for Achieving the Object]

The inventor of the present invention conducted research in order to achieve the aforementioned object. As a result, the inventor found that ceramide NH having an acyl chain length of C18 can significantly improve skin condition as compared with ceramide NHs having other acyl chain lengths, and accomplished the present invention.

That is, the present invention can be embodied, for example, as follows.
[1] A composition for improving condition of a body surface of an organism, the composition comprising the ingredient (X) shown below:
   (X) ceramide NH comprising a C18 acyl chain.
[2] The composition mentioned above, wherein the body surface is skin.
[3] The composition mentioned above, wherein the composition is for improving a lamellar structure of skin, improving a barrier function of skin, or improving a moisturizing capacity of skin.
[4] The composition mentioned above, wherein the composition is for preventing and/or treating a symptom relating to deterioration in skin condition.
[5] The composition mentioned above, wherein the symptom is rough skin, dry skin, squama (scale), desquamation, dermatitis, or psoriasis.
[6] The composition mentioned above, wherein the composition is a cosmetic composition or a pharmaceutical composition.
[7] The composition mentioned above, wherein the composition further contains an ingredient for a cosmetic or pharmaceutical.
[8] The composition mentioned above, wherein the ingredient for a cosmetic or pharmaceutical is selected from the group consisting of binders, disintegrants, lubricants, stabilizers, diluents, surfactants, pH adjusters, vitamins, minerals, perfumes, pigments, preservatives, terpenoids, lipids, fatty acids, alcohols, water, antioxidants, anti-inflammatories, moisturizing ingredients, anti-ageing ingredients, anti-cellulite ingredients, skin whitening ingredients, skin tanning ingredients, UV filters, and combinations thereof.
[9] The composition mentioned above, wherein at least a terpenoid, a lipid, a fatty acid, or a combination thereof is selected.
[10] The composition mentioned above, wherein the terpenoid(s) is/are selected from the group consisting of cholesterol, cholesterol sulfate, squalene, pristane, and combinations thereof.
[11] The composition mentioned above, wherein the lipid(s) is/are selected from the group consisting of triolein, phosphatidylethanolamine, and combinations thereof.
[12] The composition mentioned above, wherein the fatty acid(s) is/are selected from the group consisting of lauric acid, myristic acid, palmitic acid, stearic acid, arachidic acid, behenic acid, lignoceric acid, oleic acid, linolenic acid, and combinations thereof.
[13] The composition mentioned above, wherein the ingredient (X) comprises ceramide NH comprising a C18:1 alkyl chain and a C18 acyl chain.
[14] The composition mentioned above, wherein the ingredient (X) comprises ceramide NH comprising a C18:1 alkyl chain and a C18:0 acyl chain.
[15] The composition mentioned above, wherein the C18:1 alkyl chain has hydroxyl groups at positions C1, C3, and C6 but has no additional hydroxyl group at position other than C1, C3, and C6.

### [Brief Description of Drawings]

[Fig. 1]
   A photograph showing Maltese cross patterns of C14-CeramideNH (ceramide NH having an acyl chain length of C14).
[Fig. 2]
   A photograph showing Maltese cross patterns of C16-CeramideNH (ceramide NH having an acyl chain length of C16).
[Fig. 3]
   A photograph showing Maltese cross patterns of C18-CeramideNH (ceramide NH having an acyl chain length of C18).
[Fig. 4]
   A photograph showing Maltese cross patterns of C22-CeramideNH (ceramide NH having an acyl chain length of C22).
[Fig. 5]
   A diagram showing effects of recovering skin condition by ceramide NHs having different acyl chain lengths. C14, C16, C18, and C22 represent acyl chain lengths of ceramide NHs.

### [Modes for Carrying out the Invention]

Hereafter, the present invention will be explained in detail. The explanations of the present invention below may be used independently or in any appropriate combination, unless otherwise stated.

### <1> Composition of the present invention

The composition of the present invention is a composition containing ceramide NH having a length of acyl chain (an acyl chain length) of C18. This ceramide NH is referred to as "active ingredient".

The composition of the present invention may be, for example, a cosmetic composition or a pharmaceutical composition. The composition of the present invention may particularly be a cosmetic composition. A cosmetic composition refers to a composition which is used for cosmetic use, and it may be synonymous with a cosmetic. A pharmaceutical composition refers to a composition which is used for pharmaceutical use, and it may be synonymous with a pharmaceutical. Both the terms "cosmetic composition" and "pharmaceutical composition" may include quasi drugs such as medicinal cosmetics, unless otherwise stated.

The composition of the present invention can be used by, for example, applying the same to an organism. The phrase "application of the composition of the present invention to an organism" may mean that the composition of the present invention is brought into contact with a desired portion of an organism so that a desired effect is obtained. The composition of the present invention can be applied to an organism in a manner described in the method of the present invention described below. The application target and application portion of the composition of the present invention can be appropriately selected according to various conditions such as use purpose of the composition of the present invention. Examples of the organism (namely, the application target of the composition of the present invention) include mammals. Examples of the mammals include primates such as human, monkey, and chimpanzee; rodents such as mouse, rat, hamster, and guinea pig; and other various mammals such as rabbit, horse, cow, sheep, goat, pig, dog, and cat. Particular examples of mammals include human. Examples of the application portion of the composition of the present invention include a body surface of an organism. Examples of the body surface include skin and body hair. Particular examples of the body surface include skin. The skin may be any part of skin. Examples of the skin include skin of face, head, and body. Examples of body include hands, arms, foots, legs, shoulders, breasts, waist, hip, back, and any other parts of body. The body hair may be any part of body hair. Examples of the body hair include head hair.

The organism may be a healthy individual or may be an unhealthy individual. The organism may or may not be, for example, an individual for which deterioration in condition of a body surface, such as skin and body hair, is observed. The organism may or may not be, specifically, for example, an individual having a symptom relating to deterioration in condition of a body surface, such as skin and body hair.

The application portion of the composition of the present invention may be a portion in healthy state or may be a portion in unhealthy state. The application portion of the composition of the present invention may or may not be, for example, a body surface, such as skin and body hair, for which deterioration in condition is observed. The application portion of the composition of the present invention may or may not be, specifically, for example, a body surface, such as skin and body hair, having a symptom relating to deterioration in condition.

Examples of the "desired effect" in use of the composition of the present invention include an effect obtainable by use, for example, application to an organism, of the active ingredient. That is, the effect obtainable by use, for example, application to an organism, of the active ingredient may be obtained by use, for example, application to an organism, of the composition of the present invention. In other words, the composition of the present invention may have a function of providing the effect obtainable by use, for example, application to an organism, of the active ingredient. The aforementioned descriptions concerning use, for example, application to an organism, of the composition of the present invention can be applied similarly to use, for example, application to an organism, of the active ingredient.

By using the active ingredient, specifically, by applying the active ingredient to an organism, condition of the application portion of the active ingredient may be improved in the organism, namely, an effect of improving condition of the application portion of the active ingredient may be obtained in the organism. Hence, by using the composition of the present invention, specifically, by applying the composition of the present invention to an organism, condition of the application portion of the active ingredient (specifically, the application portion of the composition of the present invention) may be improved in the organism, namely, an effect of improving condition of the application portion of the active ingredient (specifically, the application portion of the composition of the present invention) may be obtained in the organism. In other words, the composition of the present invention has a function of improving condition of the application portion of the active ingredient (specifically, the application portion of the composition of the present invention). Hence, the composition of the present invention may be a composition for improving condition of the application portion thereof, such as a cosmetic or pharmaceutical composition for improving condition of the application portion thereof.

Examples of the improvement in condition include improvement in condition of a body surface, such as skin and body hair. Particular examples of the improvement in condition include improvement in skin condition. Hence, the composition of the present invention may be a composition for improving condition of a body surface, such as skin and body hair, for example, a cosmetic or pharmaceutical composition for improving condition of a body surface, such as skin and body hair. The composition of the present invention may be, particularly, a composition for improving skin condition, for example, a cosmetic or pharmaceutical composition for improving skin condition.

Examples of improvement in skin condition include improvement in a lamellar structure of skin, such as increase in orderliness of lamellar structure; improvement, such as increase, in a barrier function of skin; and improvement, such as increase, in a moisturizing capacity of skin. Hence, the composition of the present invention may be a composition for improving the lamellar structure of skin, improving the barrier function of skin, or improving the moisturizing capacity of skin, such as a cosmetic or pharmaceutical composition for improving the lamellar structure of skin, improving the barrier function of skin, or improving the moisturizing capacity of skin. Only one of these indicators may be improved, or two or more of these indicators may be improved. Improvement in a certain indicator may improve another indicator. For example, improvement in the lamellar structure may improve a function of skin, such as the barrier function of skin and the moisturizing capacity of skin. In other words, a composition for improving the barrier function of skin or improving the moisturizing capacity of skin may be an embodiment of a composition for improving the lamellar structure of skin. Also, for example, improvement in the barrier function of skin may improve the moisturizing capacity of skin. In other words, a composition for improving the moisturizing capacity of skin may be an embodiment of a composition for improving the barrier function of skin. Specific examples of skin condition include condition of stratum corneum of skin. That is, improvement in the lamellar structure of skin may specifically be improvement in the lamellar structure of stratum corneum of skin. Improvement in the barrier function of skin may specifically be improvement in the barrier function of stratum corneum of skin. Improvement in the moisturizing capacity of skin may specifically be improvement in the moisturizing capacity of stratum corneum of skin.

Methods for measuring improvement in condition of a body surface, such as skin and body hair, are not particularly limited. Improvement in condition of a body surface can be measured by using a parameter reflecting the condition of the body surface as an indicator. For example, examples of parameters reflecting skin condition include transepidermal water loss (TEWL) and order parameter S. That is, improvement in skin condition can be measured as, for example, a decrease in TEWL. TEWL can be measured in a conventional manner. A decrease in TEWL may mean, specifically, for example, improvement in the barrier function of skin. A decrease in TEWL may also mean, specifically, for example, improvement in the moisturizing capacity of skin. Improvement in skin condition can also be measured as, for example, an increase in the order parameter S. The order parameter S can be measured in a conventional manner. The order parameter S can be measured by, for example, the Electron Spin Resonance (ESR) method. The order parameter S can be measured, specifically, for example, as described in the Examples section. The order parameter S represents orderliness of array of fatty acid chains, such as orderliness of lamellar structure, and a higher value of the order parameter S indicates that fatty acid chains are arrayed in a more orderly manner. An increase in the order parameter S may mean, specifically, for example, improvement in the lamellar structure of skin.

It is sufficient that such indicators as exemplified above each are improved when using the active ingredient (specifically, when using the composition of the present invention) as compared with when not using the active ingredient (specifically, when not using the composition of the present invention). The term "improvement" used for each of such indicators as exemplified above in case that deterioration in this indicator occurs when not using the active ingredient (specifically, when not using the composition of the present invention) shall include a reduction of such deterioration when using the active ingredient (specifically, when using the composition of the present invention).

By using the active ingredient, specifically, by applying the active ingredient to an organism, an effect based on improvement in condition of the application portion of the active ingredient may be obtained in the organism. It is expected that improvement in condition of the application portion of the active ingredient enables preventing and/or treating a symptom relating to deterioration in condition of this portion. That is, examples of the effect based on improvement in condition of the application portion of the active ingredient include an effect of preventing and/or treating a symptom relating to deterioration in condition of this portion. The symptom relating to deterioration in condition of the application portion of the active ingredient may or may not be a disease. Examples of the symptom relating to deterioration in condition of the application portion of the active ingredient include a symptom caused by deterioration in condition of this portion and a symptom accompanied by deterioration in condition of this portion. Hence, the composition of the present invention may be a composition for preventing and/or treating a symptom relating to deterioration in condition of the application portion thereof, such as a cosmetic or pharmaceutical composition for preventing and/or treating a symptom relating to deterioration in condition of the application portion thereof. The composition for preventing and/or treating a symptom relating to deterioration in condition of the application portion thereof may be an embodiment of the composition for improving condition of the application portion thereof.

Examples of the deterioration in condition include deterioration in condition of a body surface, such as skin and body hair. Particular examples of the deterioration in condition include deterioration in skin condition. Hence, the composition of the present invention may be a composition for preventing and/or treating a symptom relating to deterioration in condition of a body surface, such as skin and body hair, for example, a cosmetic or pharmaceutical composition for preventing and/or treating a symptom relating to deterioration in condition of a body surface, such as skin and body hair. The composition of the present invention may be, particularly, a composition for preventing and/or treating a symptom relating to deterioration in skin condition, such as a cosmetic or pharmaceutical composition for preventing and/or treating a symptom relating to deterioration in skin condition.

Examples of the deterioration in skin condition include deterioration in the lamellar structure of skin, such as decrease in orderliness of lamellar structure; deterioration, such as decrease, in the barrier function of skin; and deterioration, such as decrease, in the moisturizing capacity of skin. Examples of the symptom relating to deterioration in skin condition include rough skin, dry skin, squama (scale), desquamation, dermatitis, and psoriasis.

The active ingredient, specifically, the composition of the present invention, may be used for therapeutic purpose, or may be used for non-therapeutic purpose. That is, such effects as exemplified above each may be obtained for therapeutic purpose or non-therapeutic purpose, unless otherwise stated. In the case of non-therapeutic purpose, the phrase "treatment of a symptom" may be interpreted as "improvement of a symptom". The term "therapeutic purpose" may mean, for example, that an act on a human body for treatment is included, and particularly, may mean that an act is carried out as a medical act. The term "non-therapeutic purpose" may mean, for example, that an act on a human body for treatment is not included, and particularly, may mean that an act is carried out as a non-medical act. Examples of the non-therapeutic purpose include such purposes as those for health promotion and beauty.

Ceramide NH is a ceramide of 6-hydroxysphingosine (6HS). Ceramide NH may also be referred to as, for example, "ceramide 8". Each variation of ceramide NH is also referred to as "ceramide NH species". Each variation of 6HS is also referred to as "6HS species".

The term "6HS" refers to a long-chain amino alcohol referred to as a sphingoid base, which has such a structure as described below. 6HS includes an alkyl chain, wherein the alkyl chain has an amino group at position C2. That is, the carbon present at either one terminus of the alkyl chain and linked to the aminated carbon (position C2) is regarded as position C1 of the alkyl chain. The alkyl chain has a hydroxyl group at C6 and further has one or more hydroxyl groups. The alkyl chain may have hydroxyl groups, for example, at positions C1, C3, and C6. The alkyl chain may or may not have additional hydroxyl group(s) other than the hydroxyl groups at positions C1, C3, and C6. The alkyl chain may typically have no additional hydroxyl group other than the hydroxyl groups at C1, C3, and C6. The length and the degree of unsaturation of the alkyl chain may vary. The length of alkyl chain (alkyl chain length) may be, for example, C14 to C26, such as C14, C16, C18, C20, C22, C24, and C26. The alkyl chain length may be, for example, particularly, C16, C18, or C20. The alkyl chain length may be, for example, more particularly, C18. The alkyl chain length may be interpreted as the carbon number, that is, the number of carbon atoms, of the alkyl chain. The alkyl chain has an unsaturated double bond at position C4 (namely, between C4 and C5). The unsaturated double bond at position C4 may be, for example, a trans double bond. The alkyl chain may or may not have an additional unsaturated double bond at position other than C4. The alkyl chain may typically have no additional unsaturated double bond at position other than C4. That is, the term "alkyl chain" used for 6HS and ceramide NH refers to one having at least one unsaturated double bond, such as alkenyl and alkadienyl chains, unless otherwise stated. The configurations of chiral centers may or may not be identical to those in the 6HS moiety of a natural ceramide NH. The position C2 may be, for example, 2S. The position C3 may be, for example, 3R. The position C4 may be, for example, 4E (namely trans double bond). The position C6 may be, for example, 6S or 6R. The position C6 may be, particularly, for example, 6R. The configurations of chiral centers may be, for example, 2S, 3R, 4E, and 6R or 2S, 3R, 4E, and 6S. The configurations of chiral centers may be, particularly, for example, 2S, 3R, 4E, and 6R. The number of carbons in the alkyl chain can be indicated as "n". 6HS having an alkyl chain whose number of carbons is "n" is also referred to as "Cn 6HS" or "Cn-alkyl 6HS". For example, the term "C18 6HS" collectively refers to 6HS species having an alkyl chain having a length of C18. The number of unsaturated double bonds of the alkyl chain can be indicated as "m". 6HS having an alkyl chain whose number of carbons is "n" and whose number of unsaturated double bonds is "m" is also referred to as "Cn:m 6HS" or "Cn:m-alkyl 6HS". Examples of 6HS can include such variant species of 6HS, wherein the variant species have different lengths and/or different degrees of unsaturation. Specific examples of 6HS include C18:1 6HS, which has a C18 alkyl chain having one unsaturated double bond. More specific examples of 6HS include C18:1 6HS that has hydroxyl groups at positions C1, C3, and C6 but has no additional hydroxyl group at position other than C1, C3, and C6. Thus, the term "6HS" may collectively refer to such variant species of 6HS, such as the 6HS species exemplified above.

The term "ceramide NH" refers to a compound having a structure in which 6HS and a fatty acid are covalently linked to each other via an amide bond. That is, ceramide NH comprises a 6HS moiety (namely, a moiety corresponding to 6HS) and a fatty acid moiety (namely, a moiety corresponding to a fatty acid), wherein the moieties are covalently linked to each other via an amide bond. The 6HS moiety can also be referred to as an "alkyl chain". The fatty acid moiety can also be referred to as an "acyl chain". The amide bond may form between the amino group at position C2 of 6HS and a carboxyl group of the fatty acid. The aforementioned descriptions concerning 6HS are similarly applicable to the 6HS moiety. That is, for example, the length and the degree of unsaturation of the alkyl chain, that is, the 6HS moiety, may vary as with those of 6HS. That is, examples of ceramide NH can include ceramides of the 6HS species exemplified above. Specific examples of ceramide NH include ceramides of C18 6HS. More specific examples of ceramide NH include ceramides of C18:1 6HS. Still more specific examples of ceramide NH include ceramides of C18:1 6HS, wherein C18:1 6HS has hydroxyl groups at positions C1, C3, and C6 but has no additional hydroxyl group at position other than C1, C3, and C6. The length and the degree of unsaturation of the acyl chain, that is, the fatty acid moiety, may also vary. The acyl chain length may be, for example, C14 to C26, such as C14, C16, C18, C20, C22, C24, and C26. The acyl chain length may be, for example, particularly, C14, C16, C18, or C22. However, the acyl chain length of the ceramide NH as the active ingredient is C18. The acyl chain length may be interpreted as the carbon number, that is, the number of carbon atoms, of the acyl chain. The acyl chain may be saturated or may be unsaturated. The acyl chain may have one or more unsaturated double bonds. The acyl chain may or may not have a functional group (namely, substituent group). The acyl chain may have one or more functional groups (substituent groups). Examples of the functional group (substituent group) include hydroxy group. However, the acyl chain has no hydroxy group at C2. The carbon constituting the amide bond (formerly, the carbon of the carboxyl group) is regarded as position C1 of the acyl chain. The acyl chain may typically have no hydroxy group. The acyl chain may typically have no functional group (substituent group). Ceramide NH having an alkyl chain whose number of carbons is "n", that is, ceramide NH comprising a Cn 6HS moiety, is also referred to as "Cn-alkyl ceramide NH". The number of carbons of the acyl chain can be indicated as "x". Ceramide NH having an acyl chain whose number of carbons is "x", that is, ceramide NH comprising a Cx acyl moiety, is also referred to as "Cx-acyl ceramide NH". The number of unsaturated double bonds of the acyl chain can be indicated as "y". Ceramide NH having an acyl chain whose number of carbons is "x" and whose number of unsaturated double bonds is "y", that is, ceramide NH comprising a Cx:y acyl moiety, is also referred to as "Cx:y-acyl ceramide NH". Ceramide NH having an alkyl chain whose number of carbons is "n" and an acyl chain whose number of carbons is "x", that is, ceramide NH comprising a Cn 6HS moiety and a Cx acyl moiety, is also referred to as "Cn-alkyl/Cx-acyl ceramide NH". For example, the term "C18-alkyl ceramide NH" collectively refers to ceramide NHs having an alkyl chain with a length of C18 and having any acyl chain. For example, the term "C18-acyl ceramide NH" collectively refers to ceramide NHs having an acyl chain with a length of C18 and having any alkyl chain. For example, the term "C18-alkyl/C18-acyl ceramide NH" collectively refers to ceramide NHs having an alkyl chain with a length of C18 and an acyl chain with a length of C18. In the case of ceramide NH having an alkyl chain whose number of carbons is "n" and whose number of unsaturated double bonds is "m", that is, ceramide NH comprising a Cn:m 6HS moiety, the term "Cn" used for the aforementioned name can be rewritten to "Cn:m". The same shall apply to "Cx" of the acyl chain. For example, the term "C18: 1-alkyl/C18:0-acyl ceramide NH" collectively refers to ceramide NHs having an alkyl chain with a length of C18 having one double bond and a saturated acyl chain with a length of C18. Ceramide NH defined with the aforementioned name may consist of a single kind of ceramide NH species or may consist of a combination of two or more kinds of ceramide NH species, unless otherwise stated. For example, Cx-acyl ceramide NH may consist of a single kind of Cx-acyl ceramide NH species or may consist of a combination of two or more kinds of Cx-acyl ceramide NH species. Such a combination may consist of two or more kinds of Cx-acyl ceramide NH species having different alkyl chains and/or different acyl chains, such as alkyl chains having different lengths and/or different degrees of unsaturation and/or acyl chains having different unsaturation degrees. Thus, the term "ceramide NH" may collectively refer to such variant species of ceramide NH, such as the ceramide NH species exemplified above.

As an example of ceramide NH, a typical structure of C18:1-alkyl ceramide NH is shown in the structural formula (I) shown below. The "n" in the formula corresponds to "x-2" in the aforementioned explanations of ceramide NH. That is, for example, this formula when "n" therein is 16 represents a typical structure of C18:1-alkyl/C18:0-acyl ceramide NH.

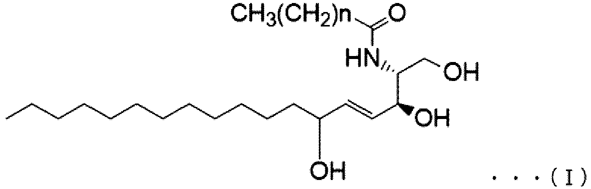

As the active ingredient, ceramide NH having an acyl chain length of C18 (C18-acyl ceramide NH) is selected. The term "C18-acyl ceramide NH" is as described above. The active ingredient (C18-acyl ceramide NH) may consist of a single kind of C18-acyl ceramide NH species or may consist of a combination of two or more kinds of C18-acyl ceramide NH species. When the active ingredient consists of a combination of two or more kinds of C18-acyl ceramide NH species, the term "amount of the active ingredient" refers to the total amount of the combination.

The active ingredient may comprise, for example, the C18-acyl ceramide NH exemplified above. The active ingredient may comprise, specifically, for example, C18-alkyl/C18-acyl ceramide NH. The active ingredient may comprise, more specifically, for example, C18:1-alkyl/C18-acyl ceramide NH. The active ingredient may comprise, still more specifically, for example, C18:1-alkyl/C18:0-acyl ceramide NH. The active ingredient may comprise, still more specifically, for example, C18:1-alkyl/C18-acyl ceramide NH, such as C18:1-alkyl/C18:0-acyl ceramide NH, wherein the alkyl chain has hydroxyl groups at positions C1, C3, and C6 but has no additional hydroxyl group at position other than C1, C3, and C6. The active ingredient may comprise, still more specifically, for example, C18:1-alkyl/C18-acyl ceramide NH, such as C18:1-alkyl/C18:0-acyl ceramide NH, wherein the acyl chain has no substituent group. The active ingredient may comprise, particularly specifically, for example, C18:1-alkyl/C18-acyl ceramide NH, such as C18:1-alkyl/C18:0-acyl ceramide NH, wherein the alkyl chain has hydroxyl groups at positions C1, C3, and C6 but has no additional hydroxyl group at position other than C1, C3, and C6, and the acyl chain has no substituent group. The phrase "the active ingredient comprises a certain C18-acyl ceramide NH" means that at least the certain C18-acyl ceramide NH is used as the active ingredient and include when the active ingredient consists of the certain C18-acyl ceramide NH and when the active ingredient consists of a combination of the certain C18-acyl ceramide NH and one or more kinds of other C18-acyl ceramide NH species. For example, phrase "the active ingredient comprises a certain C18:1-alkyl/C18-acyl ceramide NH" means that at least C18:1-alkyl/C18-acyl ceramide NH is used as the active ingredient and include when the active ingredient consists of C18:1-alkyl/C18-acyl ceramide NH and when the active ingredient consists of a combination of C18:1-alkyl/C18-acyl and one or more kinds of other C18-acyl ceramide NH species. When "the active ingredient comprises a certain C18-acyl ceramide NH", the ratio of the amount of the certain C18-acyl ceramide NH to the total amount of the active ingredient may be, for example, 30% by weight or more, 40% by weight or more, 50% by weight or more, 60% by weight or more, 70% by weight or more, 80% by weight or more, 90% by weight or more, 95% by weight or more, 97% by weight or more, 99% by weight or more, 99.5% by weight or more, 99.9% by weight or more, or 100% by weight.

As the active ingredient, a commercially available C18-acyl ceramide NH may be used, or C18-acyl ceramide NH appropriately prepared and obtained may be used. Methods for producing C18-acyl ceramide NH are not particularly limited. C18-acyl ceramide NH can be produced by, for example, known methods. C18-acyl ceramide NH can be produced by, for example, a chemical synthesis method, enzymatic method, bioconversion method, fermentation method, extraction method, or a combination of these. C18-acyl ceramide NH can be produced by, specifically, for example, conversion from 6HS.

Methods for producing 6HS are not particularly limited. 6HS can be produced by, for example, known methods. 6HS can be produced by, for example, a chemical synthesis method, enzymatic method, bioconversion method, fermentation method, extraction method, or a combination of these. 6HS can be produced by, specifically, for example, the method disclosed in FR2843589A1.

Methods for converting 6HS to C18-acyl ceramide NH are not particularly limited. 6HS can be converted to C18-acyl ceramide NH by, for example, a chemical reaction with a fatty acid (US patent 5,869,711). The chemical reaction can be carried out under, for example, typical conditions for condensing an amine and a carboxylic acid to form an amide bond. The chemical reaction can be carried out using, specifically, for example, a condensing agent. Examples of the condensing agent include carbodiimides such as water soluble carbodiimides (WSCs). Specific examples of the WSCs include 1-Ethyl-3-(3-dimethylaminopropyl)carbodiimide hydrochloride. The fatty acid is not particularly limited, so long as it provides the acyl chain of C18-acyl ceramide NH to be produced. That is, specific examples of the fatty acid include stearic acid (18:0), oleic acid (18:1), linoleic acid (18:2), and linolenic acid (18:3). Particular examples of the fatty acid include stearic acid (18:0). As 6HS, a single kind of 6HS species may be used, or two or more kinds of 6HS species may be used in combination. As the fatty acid, a single kind of fatty acid may be used, or two or more kinds of fatty acids may be used in combination. When using two or more kinds of 6HS species and/or two or more kinds of fatty acids, a mixture of two or more kinds of C18-acyl ceramide NH species is produced.

As the active ingredient, C18-acyl ceramide NH purified to a desired extent may be used, or a material containing C18-acyl ceramide NH may be used.

The composition of the present invention may consist of the active ingredient or may contain an ingredient other than the active ingredient. That is, the active ingredient may be used as the composition of the present invention as it is or may be used as the composition of the present invention in combination with another ingredient. As the ingredient other than the active ingredient, a single kind of ingredient may be used, or two or more kinds of ingredients may be used in combination.

The ingredient other than the active ingredient is not particularly limited, so long as an intended effect is obtained. As the ingredient other than the active ingredient, ingredients acceptable depending on the use purpose of the composition of the present invention can be used. Examples of the ingredient other than the active ingredient include physiologically acceptable ingredients. Examples of the physiologically acceptable ingredients include ingredients for a cosmetic or pharmaceutical, such as ingredients generally used for cosmetic use or pharmaceutical use. The term "ingredient for a cosmetic" refers to an ingredient usable as an ingredient in a cosmetic. The term "ingredient for a pharmaceutical" refers to an ingredient usable as an ingredient in a pharmaceutical. Examples of the ingredient other than the active ingredient include binders, disintegrants, lubricants, stabilizers, diluents, surfactants, pH adjusters, vitamins, minerals, perfumes, pigments, preservatives, terpenoids, lipids, fatty acids, alcohols, and water. Particular examples of the ingredient other than the active ingredient include terpenoids, lipids, and fatty acids. Examples of the terpenoids include sterols, squalene, and pristane. Examples of the sterols include cholesterol and cholesterol sulfate. Examples of the lipids include triacylglycerols and glycerophospholipids. Examples of the triacylglycerols include triolein. Examples of the glycerophospholipids include phosphatidylethanolamine. Examples of the fatty acids include lauric acid (12:0), myristic acid (14:0), palmitic acid (16:0), stearic acid (18:0), arachidic acid (20:0), behenic acid (22:0), lignoceric acid (24:0), cerotic acid (26:0), myristoleic acid (14:1), palmitoleic acid (16:1), oleic acid (18:1), linoleic acid (18:2), and linolenic acid (18:3). Particular examples of the fatty acids include lauric acid, myristic acid, palmitic acid, stearic acid, arachidic acid, behenic acid, lignoceric acid, oleic acid, and linolenic acid. More particular examples of the fatty acids include lauric acid, myristic acid, palmitic acid, stearic acid, oleic acid, and linolenic acid. More particular examples of the fatty acids include lauric acid. Specific examples of the ingredient other than the active ingredient also include antioxidants, anti-inflammatories, moisturizing ingredients, anti-ageing ingredients, anti-cellulite ingredients, skin whitening ingredients, skin tanning ingredients, and UV filters. Specific examples of the ingredient other than the active ingredient also include ceramides such as ceramide NHs not selected as the active ingredient. As the ingredient other than the active ingredient, for example, one or more ingredients exemplified above may be at least selected. In other words, the ingredient other than the active ingredient may comprise one or more ingredients exemplified above. The phrase "a certain ingredient is selected as the ingredient other than the active ingredient" or "the ingredient other than the active ingredient comprises a certain ingredient" means that at least the certain ingredient is used as the ingredient other than the active ingredient and includes when the ingredient other than the active ingredient consists of the certain ingredient and when the ingredient other than the active ingredient consists of a combination of the certain ingredient and one or more kinds of other ingredients. All such ingredients as exemplified above can be used as, for example, ingredients for both a cosmetic and a pharmaceutical.

When the ingredient other than the active ingredient can form a salt, the ingredient other than the active ingredient may be a free compound, a salt thereof, or a combination thereof. For example, examples of salts for acidic groups such as carboxyl group (for example, salts of fatty acids) include ammonium salt; salts with alkaline metal such as sodium and potassium; salts with alkaline earth metal such as calcium and magnesium; and salts with other metals such as aluminum and zinc.

The form of the composition of the present invention is not particularly limited, so long as an intended effect is obtained. The form of the composition of the present invention may be set according to various conditions such as the type of the active ingredient, the type(s) of the other ingredient(s), the amount(s) of the ingredient(s), the use purpose of the composition of the present invention, and the use mode of the composition of the present invention. The composition of the present invention may be formulated to an intended form. The composition of the present invention may be provided, for example, in the form of any cosmetic or pharmaceutical composition applicable to skin. Examples of the cosmetic or pharmaceutical composition, especially, examples of the cosmetic composition, include skin care cosmetics, makeup cosmetics, point makeup cosmetics, hair care products, hair setting agents, hair coloring agents, decolorizing agents, permanent agents, wave agents, body care cosmetics, UV care cosmetics. Examples of the skin care cosmetics include cleansing cosmetics (such as face washes and makeup removers), lotions, serums, packs, massage creams, milky lotions, creams, and essences. Examples of the makeup cosmetics include foundations, concealers, face powders, and make-up bases. Examples of the point makeup cosmetics include lipsticks, blushers, and eye makeups (such as eyeshadows, eyeliners, mascaras, and eyebrows). Examples of the hair care products include shampoos and conditioners (also referred to as rinse or treatment). A hair care product may be a product for, for example, obtaining such an effect as exemplified above in head hair and/or head skin. Examples of the hair setting agents include hair creams, hair waxes, hair liquids, and hair sprays. Examples of the hair coloring agents or decolorizing agents include hair manicures and hair colors. Examples of the body care cosmetics include body creams, soaps, body soaps, hand soaps, and antiperspirants. Examples of the UV care cosmetics include sunscreen cosmetics, suntan cosmetics, self-tanning cosmetics, and after-sun cosmetics. Examples of the cosmetic or pharmaceutical composition, especially, examples of the pharmaceutical composition, include external preparations, such as external skin preparations. Examples of the external preparations include solid agents (such as powders), liquid agents (such as lotions and liniments), sprays, ointments, creams, gels, and patches (such as tapes and poultices). The composition of the present invention may be provided in a form applicable to skin as it is or may be prepared in a form applicable to skin before use.

The contained amount(s) of ingredient(s) (namely, the active ingredient and optionally other ingredient(s)) in the composition of the present invention is/are not particularly limited, so long as an intended effect is obtained. The contained amount(s) of ingredient(s) in the composition of the present invention can be appropriately set according to various conditions such as the type of the active ingredient, the type(s) of the other ingredient(s), the form of the composition of the present invention, the use purpose of the composition of the present invention, and the use mode of the composition of the present invention.

The contained amount of the active ingredient in the composition of the present invention, for example, may be 0.01% by weight or more, 0.1% by weight or more, 1% by weight or more, 5% by weight or more, 10% by weight or more, 15% by weight or more, 20% by weight or more, or 30% by weight or more, may be 100% by weight or less, 99.9% by weight or less, 70% by weight or less, 50% by weight or less, 30% by weight or less, 20% by weight or less, 15% by weight or less, 10% by weight or less, 5% by weight or less, or 1% by weight or less, or may be within a range defined as a non-contradictory combination thereof. The total contained amount of the active ingredient in the composition of the present invention may specifically be, for example, 0.01 to 1% by weight, 1 to 5% by weight, 5 to 10% by weight, 10 to 20% by weight, 20 to 30% by weight, or 30 to 50% by weight. The total contained amount of the active ingredient in the composition of the present invention may specifically be, for example, 0.01 to 50% by weight, 0.01 to 30% by weight, 0.01 to 10% by weight, or 0.01 to 5% by weight. The total contained amount of the active ingredient in the composition of the present invention may specifically be, for example, 1 to 50% by weight, 1 to 30% by weight, 1 to 10% by weight, or 1 to 5% by weight. The total contained amount of the active ingredient in the composition of the present invention may specifically be, for example, 5 to 50% by weight, 5 to 30% by weight, or 5 to 10% by weight.

When the composition of the present invention contains ceramide(s) other than the active ingredient, the ratio of the contained amount of the active ingredient to the total contained amount of ceramides in the composition of the present invention may be, for example, 30% by weight or more, 40% by weight or more, 50% by weight or more, 60% by weight or more, 70% by weight or more, 80% by weight or more, 90% by weight or more, 95% by weight or more, 97% by weight or more, 99% by weight or more, 99.5% by weight or more, or 99.9% by weight or more.

Furthermore, the contained amount of the active ingredient in the composition of the present invention may be, for example, such an amount that the application amount of the active ingredient is within a desired range when applying the composition of the present invention to a body surface, such as skin or body hair.

Incidentally, the contained amount of active ingredient when using a material containing the active ingredient should be calculated based on the amount of the active ingredient itself in the material.

### <2> Method of the present invention

The method of the present invention is a method comprising a step of applying the active ingredient (namely ceramide NH having an acyl chain length of C18) to an organism.

By the method of the present invention, specifically, by applying the active ingredient to an organism, condition of the application portion of the active ingredient can be improved in the organism, that is, an effect of improving condition of the application portion of the active ingredient can be obtained in the organism. Hence, the method of the present invention may be a method for improving condition of the application portion of the active ingredient.

Improvement in condition is as described above. That is, the method of the present invention may be a method for improving condition of a body surface, such as skin and body hair. The method of the present invention may be, particularly, a method for improving skin condition. The method of the present invention may be a method for improving the lamellar structure of skin, improving the barrier function of skin, or improving the moisturizing capacity of skin. A method for improving the barrier function of skin or improving the moisturizing capacity of skin may be an embodiment of a method for improving the lamellar structure of skin. A method for improving the moisturizing capacity of skin may be an embodiment of a method for improving the barrier function of skin.

By the method of the present invention, specifically, by applying the active ingredient to an organism, an effect based on improvement in condition of the application portion of the active ingredient may be obtained in the organism. Hence, the method of the present invention may be a method for obtaining an effect based on improvement in condition of the application portion of the active ingredient.

The effect based on improvement in condition of the application portion of the active ingredient is as described above. That is, the method of the present invention may be a method for preventing and/or treating a symptom relating to deterioration in condition of the application portion of the active ingredient. The method of the present invention may be a method for preventing and/or treating a symptom relating to deterioration in condition of a body surface, such as skin and body hair. The method of the present invention may be, particularly, a method for preventing and/or treating a symptom relating to deterioration in skin condition.

The application scheme (for example, application target, application portion, application timing, application period, application number of times, application amount, and other conditions relating to application) of the active ingredient is not particularly limited, so long as an intended effect is obtained. The application scheme of the active ingredient can be appropriately set according to various conditions such as the type of the active ingredient, the type, age, and health condition of the application target, and the use purpose of the active ingredient.

The active ingredient, for example, may be applied to an organism as it as or may be applied to an organism after being prepared as a composition, such as a cosmetic composition and a pharmaceutical composition, containing the active ingredient. For the composition containing the active ingredient, the descriptions concerning the composition of the present invention can be similarly applied. In addition, the active ingredient may be applied solely or in combination with another ingredient. For the other ingredient, the descriptions concerning the ingredient other than the active ingredient in the composition of the present invention can be similarly applied. In addition, examples of the other ingredient also include compositions such as cosmetic compositions and pharmaceutical compositions.

The application target and application portion of the active ingredient are as described above. That is, the active ingredient can be applied to, for example, such an application target as exemplified above. The active ingredient can be applied to, for example, such an application portion as exemplified above. The active ingredient can be applied to the organism by an appropriate means such as, for example, spreading, pasting, and spraying.

The application period of the active ingredient may be, for example, 1 hour or longer, 6 hours or longer, 1 day or longer, 3 days or longer, 1 week or longer, 4 weeks or longer, 2 months or longer, 6 months or longer, 12 months or longer, 2 years or longer, 5 years or longer, or 10 years or longer. The active ingredient may be applied, for example, routinely or only during a specific period. The active ingredient may be applied, for example, continuously or intermittently. The active ingredient may be applied, for example, until an intended effect is obtained. The active ingredient may be applied, for example, once a day, or two or more times a day as divided portions. The active ingredient may be applied, for example, every day, or once in several days. The application amount of the active ingredient at the time of each application may or may not be constant. The application amount of the active ingredient for each application, for example, may be 0.1 µg/cm² or more, 0.5 µg/cm² or more, 1 µg/cm² or more, 5 µg/cm² or more, or 10 µg/cm² or more, may be 500 mg/cm² or less, 100 mg/cm² or less, 50 mg/cm² or less, 10 mg/cm² or less, 5 mg/cm² or less, 1 mg/cm² or less, 0.5 mg/cm² or less, or 0.1 mg/cm² or less, or may be within a range defined as a combination thereof. The application amount of the active ingredient for each application may specifically be, for example, 0.1 µg/cm² to 500 mg/cm².

The active ingredient can be applied to an organism, for example, by using the composition of the present invention, that is, by applying the composition of the present invention. That is, an embodiment of the method of the present invention may be a method comprising a step of applying the composition of the present invention to an organism. That is, the term "application of the active ingredient" also includes application of the composition of the present invention. The application scheme (for example, application target, application portion, application timing, application period, application number of times, application amount, and other conditions relating to application) of the composition of the present invention is not particularly limited, so long as an intended effect is obtained. The application scheme of the composition of the present invention can be appropriately set according to various conditions such as the type and contained amount of the active ingredient, the type(s) and contained amount(s) of other ingredient(s), the type and form (dosage form) of the composition, the type, age, and health condition of the application target, and the use purpose of the composition of the present invention. The aforementioned descriptions concerning the application scheme of the active ingredient can be similarly applied to cases of applying the composition of the present invention to an organism. That is, the composition of the present invention can be applied to, for example, such an application target as exemplified above. The composition of the present invention can be applied to, for example, such an application portion as exemplified above. Furthermore, the application amount of the composition of the present invention can be set to an amount to that such an application amount of the active ingredient as exemplified above is obtained. In addition, the composition of the present invention may be used solely or in combination with another ingredient. For the other ingredient, the descriptions concerning the ingredient other than the active ingredient in the composition of the present invention can be similarly applied. In addition, examples of the other ingredient also include compositions such as cosmetic compositions and pharmaceutical compositions.

### <3> Use of the active ingredient

The present invention also discloses use of the active ingredient for the use purpose exemplified above. That is, the present invention discloses, for example, use of the active ingredient for improving condition of the application portion of the active ingredient (for example, the application portion of the composition of the present invention) and use of the active ingredient in manufacture of a composition (for example, a cosmetic or pharmaceutical composition) for improving condition of the application portion thereof.

The present invention also discloses the active ingredient for use in the use purpose exemplified above. That is, the present invention discloses, for example, the active ingredient for use in improving condition of the application portion of the active ingredient (for example, the application portion of the composition of the present invention) and the active ingredient for use in manufacture of a composition (for example, a cosmetic or pharmaceutical composition) for improving condition of the application portion thereof.

### [Examples]

The present invention will be more specifically explained with reference to the following non-limiting examples.

### Example 1: Evaluation of lamellar structure-forming ability

### <Preparation of samples>

Four ceramide NHs (Table 1) shown in the structural formula (I) shown below wherein n is 12, 14, 16, or 20 (namely, having an acyl chain length of C14, C16, C18, or C22) were purchased from Tokyo Chemical Industry Co., Ltd.

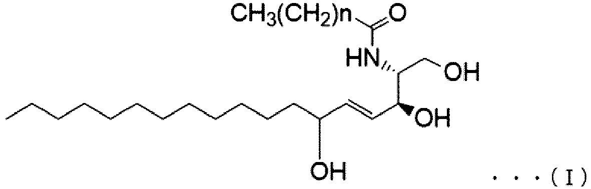

### [Table 1]

**Table 1: Ceramide NHs usen in Examples**

| Name | C14-CeramideNH | C16-CeramideNH | C18-CeramideNH | C22-CeramideNH |
|---|---|---|---|---|
| n | 12 | 14 | 16 | 20 |

### <Measurement of lamellar structure-forming ability>

As an indicator of the lamellar structure-forming ability, Maltese cross patterns were observed via the following procedures. Ceramide NH, cholesterol, and lauric acid were mixed in the mixing ratios shown in Table 2, to prepare samples. Each sample was placed in a glass tube and heated to 90°C in a thermo shaker (Thermo Shaker for Microtubes, TS-100, Biosan), to melt the entire sample. The melted sample was put to a microscope slide, and a cover slip was placed thereon. The sample was melted again on a hot plate (C-MAGHP4, IKA Japan) at 100°C, set on a polarizing microscope (DX51-P, Olympus), and Maltese cross patterns were observed after the sample had solidified. Observed images were captured with a 20x objective lens and printed on A4 size paper. The number of Maltese cross patterns observed in a 200 µm x 200 µm area was visually counted.

### <Results>

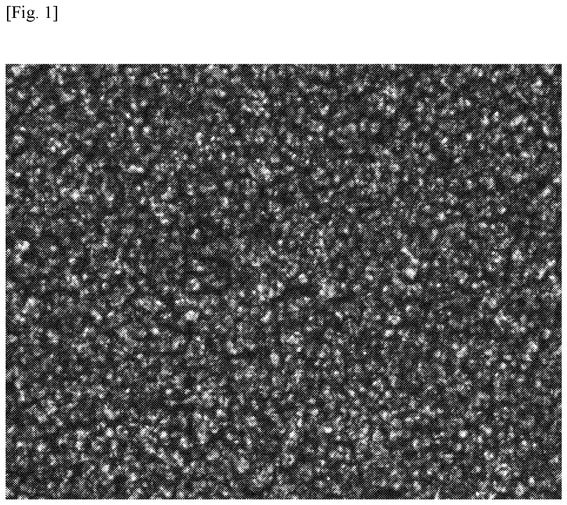

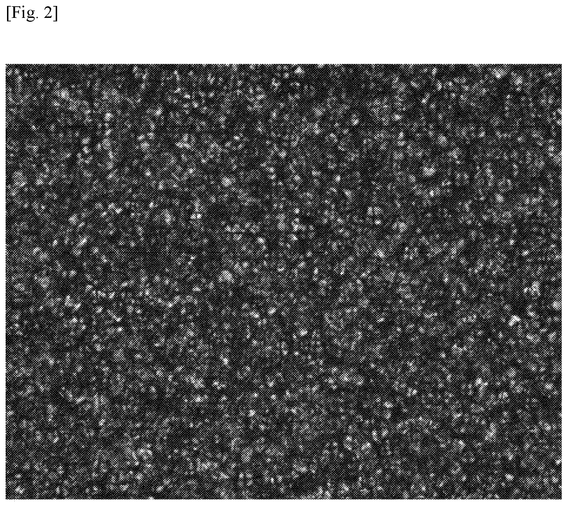

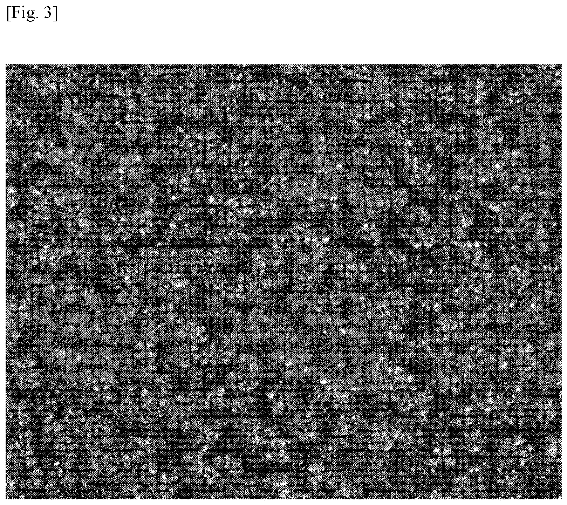

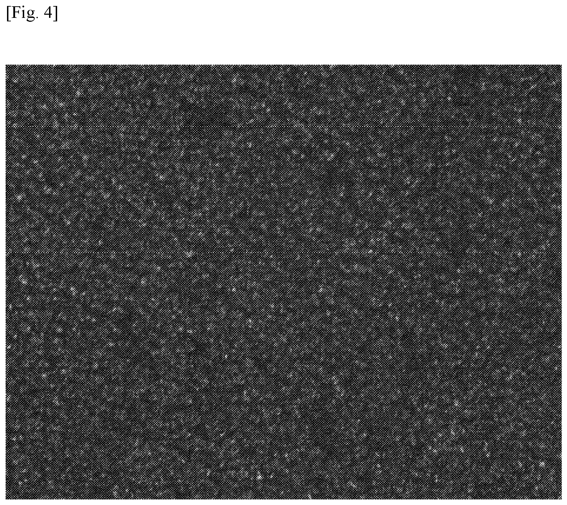

The results of observation of Maltese cross patterns are shown in Table 2 and Figures 1 to 4. Ceramide NH having an acyl chain length of C18 (C18-CeramideNH) showed a larger number of Maltese cross patterns (namely, a higher lamellar structure-forming ability) than ceramide NHs having other acyl chain lengths.

### [Table 2]

**Table 2: Number of Maltese cross patterns**

| Ceramide NH | | Cholesterol | Lauric acid | Number of Maltese cross patterns |
|---|---|---|---|---|
| | [mg] | [mg] | [mg] | |
| C14-CeramideNH | 10 | 10.09 | 10.1 | 0 |
| C16-CeramideNH | 10.01 | 10.07 | 10.06 | 21 |
| C18-CeramideNH | 10.01 | 10.05 | 10.03 | 137 |
| C22-CeramideNH | 10.01 | 10.06 | 10.07 | 0 |

### Example 2: Evaluation of effect of recovering skin condition

### <Preparation of evaluation samples>

Evaluation samples were obtained by mixing 18 wt% ceramide NH, 7 wt% squalene, 25 wt% triolein, 2 wt% cholesterol sulfate, 14 wt% cholesterol, 5 wt% phosphatidylethanolamine, 4 wt% pristane, and 25 wt% a mixture of fatty acids and fatty acid salts. The mixture of fatty acids and fatty acid salts was prepared by mixing oleic acid (35 wt%), myristic acid (15 wt%), palmitic acid (36 wt%), stearic acid (10 wt%), and linolenic acid (4 wt%) and neutralized with sodium hydroxide by 41%. The mixture of fatty acids and fatty acid salts contains 30 parts by weight of water to 100 parts by weight of fatty acids.

### <Measurement of order parameter S

The order parameter S was measured, and an effect of recovering skin condition was evaluated, via the following procedures. The order parameter S represents orderliness of array of fatty acid chains, such as orderliness of lamellar structure, and a higher value of the order parameter S indicates that fatty acid chains are arrayed in a more orderly manner. That is, an increase in the order parameter S means improvement in skin condition and may specifically mean improvement in the lamellar structure of skin.

(1) Skin samples (Frozen dermatomed human skin, abdominal origin; product number TRA002; BPI) were immersed in a 0.001% 5-DSA (5-doxyl stearic acid) solution for 30 minutes at 37°C, to allow 5-DSA to penetrate the lipid membrane structure. 5-DSA is a labeling agent for ESR measurement.
(2) The skin samples were washed with PBS solution, to remove 5-DSA adhering to the outside of the skin samples.
(3) The skin samples were dried at room temperature and immersed in 5% SLS solution for 1 hour at 37°C, to induce rough skin condition.
(4) After the skin samples were dried at room temperature, the order parameter S was calculated by ESR measurement using a JES-X310 electron spin resonance system (JEOL) and used as "pre-spreading data". ESR measurement was carried out by a method of a previous report (Mizushima J. et al., Int J Pharm. 2000 Mar 20; 197(1-2): 193-202. Effect of surfactants on human stratum corneum: electron paramagnetic resonance study.), wherein the measurement conditions were Power: 10 mM, Center Field: 336 mT, Sweep: 7.5 mT, Modulation width: 0.5 mT, Scan time: 2 min, Time constant: 0.3 sec (the same shall apply hereafter).
(5) The evaluation sample was spread to the skin sample with a spatula at 3 µg/cm².
(6) The evaluation sample was wiped off from the skin sample, the skin sample was dried at room temperature, and then the order parameter S was calculated by ESR measurement and used as "post-spreading data".

As a control, the same experiment was conducted without spreading the evaluation sample.

For each evaluation sample and control, the ratio of post-spreading data to pre-spreading data was calculated and designated as the "recovery rate" of skin condition. The recovery rate for each evaluated sample as a relative value to the recovery rate for the control was calculated and used as the "recovery effect" of the skin condition. The experiment was carried out in duplicate, and the results were obtained as average values.

### <Results>

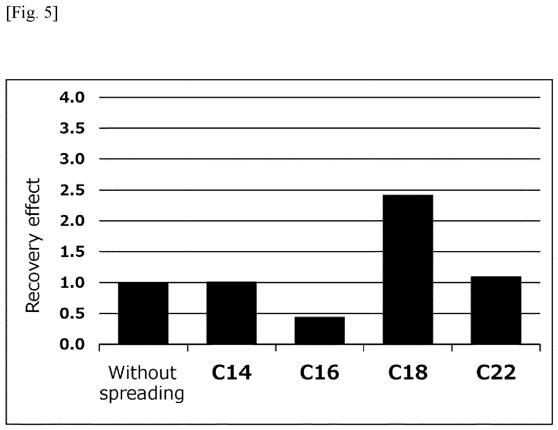

The results are shown in Figure 5. Ceramide NH having an acyl chain length of C18 (C18-CeramideNH) showed a higher recovery effect of the skin condition than ceramide NHs having other acyl chain lengths.

### [Industrial Applicability]

According to the present invention, a composition, such as a cosmetic composition, can be provided.

## Claims

1. A composition for improving condition of a body surface of an organism, the composition comprising the ingredient (X) shown below:
(X) ceramide NH comprising a C18 acyl chain.

2. The composition according to claim 1, wherein the body surface is skin.

3. The composition according to claim 1 or 2, wherein the composition is for improving a lamellar structure of skin, improving a barrier function of skin, or improving a moisturizing capacity of skin.

4. The composition according to any one of claims 1 to 3, wherein the composition is for preventing and/or treating a symptom relating to deterioration in skin condition.

5. The composition according to claim 4, wherein the symptom is rough skin, dry skin, squama (scale), desquamation, dermatitis, or psoriasis.

6. The composition according to any one of claims 1 to 5, wherein the composition is a cosmetic composition or a pharmaceutical composition.

7. The composition according to any one of claims 1 to 6, wherein the composition further contains an ingredient for a cosmetic or pharmaceutical.

8. The composition according to claim 7, wherein the ingredient for a cosmetic or pharmaceutical is selected from the group consisting of binders, disintegrants, lubricants, stabilizers, diluents, surfactants, pH adjusters, vitamins, minerals, perfumes, pigments, preservatives, terpenoids, lipids, fatty acids, alcohols, water, antioxidants, anti-inflammatories, moisturizing ingredients, anti-ageing ingredients, anti-cellulite ingredients, skin whitening ingredients, skin tanning ingredients, UV filters, and combinations thereof.

9. The composition according to claim 8, wherein at least a terpenoid, a lipid, a fatty acid, or a combination thereof is selected.

10. The composition according to claim 8 or 9, wherein the terpenoid(s) is/are selected from the group consisting of cholesterol, cholesterol sulfate, squalene, pristane, and combinations thereof.

11. The composition according to any one of claims 8 to 10, wherein the lipid(s) is/are selected from the group consisting of triolein, phosphatidylethanolamine, and combinations thereof.

12. The composition according to any one of claims 8 to 11, wherein the fatty acid(s) is/are selected from the group consisting of lauric acid, myristic acid, palmitic acid, stearic acid, arachidic acid, behenic acid, lignoceric acid, oleic acid, linolenic acid, and combinations thereof.

13. The composition according to any one of claims 1 to 12, wherein the ingredient (X) comprises ceramide NH comprising a C18:1 alkyl chain and a C18 acyl chain.

14. The composition according to any one of claims 1 to 13, wherein the ingredient (X) comprises ceramide NH comprising a C18:1 alkyl chain and a C18:0 acyl chain.

15. The composition according to claim 13 or 14, wherein the C18:1 alkyl chain has hydroxyl groups at positions C1, C3, and C6 but has no additional hydroxyl group at position other than C1, C3, and C6.
